(19) Europäisches Patentamt
European Patent Office
Office européen des brevets

(11) Numéro de publication : **0 354 835 B1**

(12) # FASCICULE DE BREVET EUROPEEN

(45) Date de publication du fascicule du brevet : **14.10.92 Bulletin 92/42**

(51) Int. Cl.$^5$ : **C07C 323/25,** A61K 7/11

(21) Numéro de dépôt : **89402209.4**

(22) Date de dépôt : **03.08.89**

(54) **N-(mercaptoalkyl)oméga-hydroxyalkylamides et leur utilisation en tant qu'agents réducteurs dans un procédé de déformation permanente des cheveux.**

(30) Priorité : **04.08.88 LU 87310**

(43) Date de publication de la demande : **14.02.90 Bulletin 90/07**

(45) Mention de la délivrance du brevet : **14.10.92 Bulletin 92/42**

(84) Etats contractants désignés : **AT BE CH DE ES FR GB GR IT LI NL SE**

(56) Documents cités :
**FR-A- 2 587 030**
**US-A- 3 768 490**

(73) Titulaire : **L'OREAL**
**14, Rue Royale**
**F-75008 Paris (FR)**

(72) Inventeur : **Maignan, Jean**
**8, Rue Halévy**
**F-93290 Tremblay Les Gonesse (FR)**
Inventeur : **Lang, Gérard**
**44, Avenue Lacour**
**F-95210 Saint Gratien (FR)**
Inventeur : **Malle, Gérard**
**18, Grande Rue**
**F-77580 Villiers Sur Morin (FR)**

(74) Mandataire : **Stalla-Bourdillon, Bernard et al**
**CABINET NONY & CIE 29, rue Cambacérès**
**F-75008 Paris (FR)**

**EP 0 354 835 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux N-(mercaptoalkyl)ω-hydroxyalkylamides et leur utilisation, en tant qu'agents réducteurs, dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou décrépage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un mercaptan.

Parmi ces derniers, ceux couramment utilisés sont l'acide thioglycolique et l'acide thiolactique ou un mélange de ces acides ainsi que leurs esters par exemple le monothioglycolate de glycérol ou de glycol.

Ces agents réducteurs sont particulièrement efficaces pour réduire les liaisons disulfures de la kératine notamment l'acide thioglycolique qui peut être considéré comme le produit de référence en permanente, et qui conduit à un taux de réduction d'environ 50%.

Ces agents réducteurs présentent cependant un inconvénient majeur dans la mesure où ils dégagent de mauvaises odeurs, d'ailleurs propres aux composés soufrés qui rendent les opérations de permanente parfois pénibles non seulement pour les personnes qui les subissent mais également pour les personnes qui les effectuent.

En vue de pallier à cet inconvénient, il est généralement fait usage d'un parfum permettant de masquer les odeurs.

Des études ont par ailleurs été conduites en vue de mettre au point de nouveaux agents réducteurs sans odeur. Ainsi, dans la demande de brevet japonais (KOKAI) n°260.058/86, il a été proposé l'utilisation de N-mercaptoalkylgluconamides qui sont des composés solubles dans l'eau, de faible volatilité et pratiquement dénués d'odeur.

Ces N-mercaptoalkylgluconamides sont obtenus par réaction de la glucono- δ -lactone avec un aminoalcanethiol.

Ces nouveaux agents réducteurs, s'ils permettent de remédier aux inconvénients des agents réducteurs connus, ne possèdent pas toutefois d'excellentes propriétés réductrices par rapport notamment à l'acide thioglycolique.

On a maintenant constaté, de façon tout à fait surprenante, qu'en utilisant une nouvelle classe de mercaptoalkylamides, notamment des N-(mercaptoalkyl) ω-hydroxyalkylamides, il était possible de remédier aux inconvénients de ces différents agents réducteurs.

Les N-(mercaptoalkyl) ω-hydroxyalkylamides selon l'invention présentent en effet d'excellentes propriétés réductrices tout à fait comparables à celles de l'acide thioglycolique mais sont dépourvus d'odeur.

La présente invention a pour objet, à titre de produits industriels nouveaux, des N-(mercaptoalkyl)ω-hydroxyalkylamides, solubles dans l'eau, correspondant à la formule générale suivante:

$$HS-(CH_2)_n-NH-\overset{\overset{\displaystyle O}{\|}}{C}-(CH_2)_m-OH \qquad (I)$$

dans laquelle:

n est 2 ou 3, et

m est 2 à 5 inclus.

Selon une forme de réalisation préférée de l'invention n est 2 et m est 3 ou 4.

Parmi les N-(mercaptoalkyl) ω-hydroxyalkylamides de formule (I) ci-dessus on peut notamment citer les suivants:

l'hydroxy-4 N-(mercapto-2 éthyl) butyramide,
l'hydroxy-3 N-(mercapto-2 éthyl) propionamide,
l'hydroxy-5 N-(mercapto-2 éthyl) valéramide,
l'hydroxy-4 N-(mercapto-3 propyl) butyramide,
l'hydroxy-6 N-(mercapto-2 éthyl) caproamide, et

l'hydroxy-6 N-(mercapto-3 propyl) caproamide.

Parmi ceux-ci l'hydroxy-4 N-(mercapto-2 éthyl) butyramide est particulièrement préféré.

La présente invention a également pour objet le procédé de préparation des mercaptoalkylamides selon l'invention, ce procédé consistant à faire réagir une mercaptoalkylamine (1) sur une lactone (2) selon le schéma réactionnel suivant:

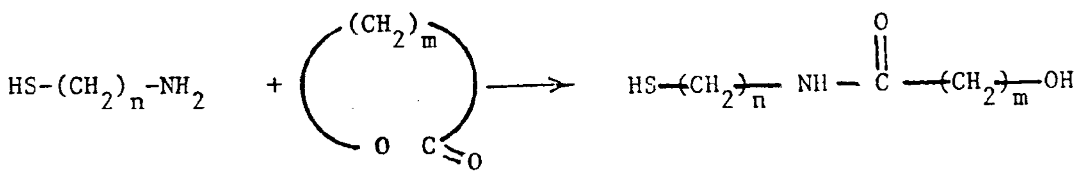

(I)

(1) (2)

Les mercaptoalkylamines (1) utilisées sont soit la mercapto-2 éthylamine ou cystéamine (n = 2) soit la mercapto-3 propylamine (n = 3).

Les lactones (2) sont la β-propionolactone (m = 2), la γ -butyrolactone (m = 3), la valérolactone (m = 4) ou la caprolactone (m = 5).

La réaction d'ouverture de la lactone est généralement conduite sous atmosphère inerte dans un alcool tel que le méthanol, l'éthanol, l'isopropanol ou le butanol, et suivant le point d'ébullition de ce dernier, à une température comprise entre 20 et 110°C.

Le temps réactionnel est généralement long et peut demander plusieurs jours de telle sorte qu'il peut être avantageux de ne pas utiliser de solvant et de mélanger dans des proportions stoechiométriques la mercaptoalkylamine et la lactone et de porter le mélange à une température comprise entre 20 et 80°C.

L'évolution de la réaction est suivie par dosage de la mercapto-alkylamine non transformée. Lorsque le temps réactionnel est trop long, il peut être avantageux d'utiliser un excès de mercaptoalkylamine qui est éliminée en fin de réaction par filtration du mélange sur une résine sulfonique acide.

Si au cours de la réaction une certaine quantité de thiol est oxydée en disulfure correspondant, le mélange réactionnel est alors dilué par deux fois son volume d'eau et agité en présence d'un mélange de résine sulfonique et de poudre de zinc pendant 3 à 10 heures.

La majorité du disulfure étant réduite, le mélange est alors filtré et on obtient une solution du composé attendu qui peut être utilisée directement.

Selon une forme préférée du procédé selon l'invention, les mercaptoalkylamines peuvent être utilisées sous forme de chlorhydrate en tant que matière première,l'amine libre étant libérée par de la triéthanolamine. Dans ce cas, après la fin de la réaction on obtient le composé attendu avec un équivalent de chlorhydrate de triéthanolamine dont la présence ne modifie pas les propriétés des N-(mercaptoalkyl) ω-hydroxyalkylamides selon l'invention.

Il peut être avantageux d'opérer dans un autoclave ce qui élimine les problèmes de sublimation de la cystéamine.

La présente invention a également pour objet à titre de produits industriels nouveaux les disulfures des composés de formule (I) ceux-ci pouvant être représentés par la formule générale suivante :

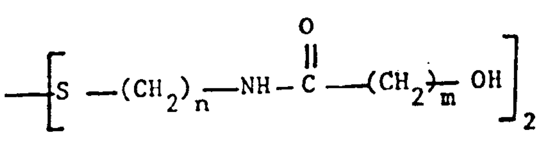

(II)

dans laquelle :

n et m ont les mêmes significations que celles données ci-dessus pour la formule (I).

Les disulfures trouvent une application dans les compositions dites "auto-neutralisantes" c'est-à-dire lorsqu'ils sont associés à un thiol, tel que par exemple un N-(mercaptoalkyl)ω-hydroxyalkylamide de formule (I) ci-dessus, dans des proportions molaires allant de 0,5 à 2,5 et de préférence de 1 à 2 (voir Brevet US 3.768.490).

Les disulfures de formules (II) selon l'invention sont obtenus par oxydation des composés de formule (I) soit à l'air, soit en utilisant par exemple de l'eau oxygénée en présence éventuellement d'ions ferreux.

Ceux-ci peuvent également être obtenus par réaction d'un disulfure de formule (3) sur une lactone (2) selon le schéma réactionnel suivant :

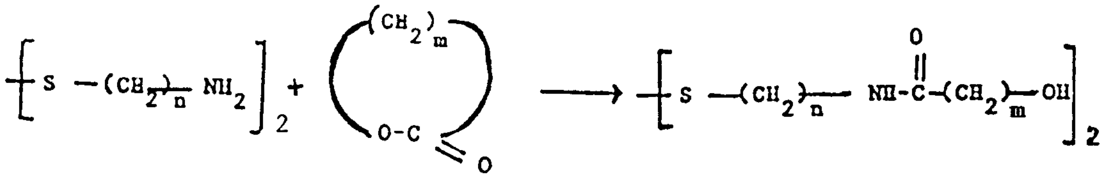

(3) (2) (II)

Parmi les disulfures de formule (II) ci-dessus on peut notamment citer les suivants :

le bis (N-éthyl hydroxy-4 butyramide) disulfure,
le bis (N-éthyl hydroxy-5 valéramide) disulfure
le bis (N-éthyl hydroxy-6 caproamide) disulfure
le bis (N-éthyl hydroxy-3 propionamide) disulfure,
le bis (N-propyl hydroxy-4 butyramide) disulfure, et
le bis (N-propyl hydroxy-6 caproamide) disulfure.

La présente invention a également pour objet une composition réductrice, pour le premier temps d'une opération de déformation permanente des cheveux, contenant dans un véhicule cosmétique approprié, au moins un N-(mercaptoalkyl) ω-hydroxyalkylamide de formule (I) telle que définie ci-dessus, en tant qu'agent réducteur.

De préférence, l'agent réducteur est présent dans la composition selon l'invention à une concentration comprise entre 2 et 20% en poids et de préférence entre 5 et 10% en poids par rapport au poids total de la composition réductrice.

Le pH de la composition est généralement compris entre 6,5 et 10, et obtenu à l'aide d'agents alcalins tels que par exemple l'ammoniaque, la monoéthanolamine, la diéthanolamine, la triéthanolamine, un carbonate ou un bicarbonate alcalin ou d'ammonium.

La composition réductrice peut également contenir divers ingrédients tels que par exemple des polymères cationiques tels que ceux utilisés dans les compositions des brevets français n°79.32078 et 80.26421 ou encore des polymères cationiques du type ionène tels que ceux utilisés dans les compositions du brevet français n°82.17364, des agents adoucissants et notamment des ammoniums quaternaires dérivés de la lanoline, des hydrolysats de protéines, des cires, des agents opacifiants, des parfums, des colorants, des tensio-actifs non-ioniques ou cationiques, des agents traitants ou encore des agents de pénétration tels que l'urée, la pyrrolidone ou la thiamorpholinone.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrépage des cheveux, la composition réductrice est de préférence sous forme d'une crème de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras etc... On peut également utiliser des liquides ou des gels contenant des agents épaississants tels que des polymères ou copolymères carboxyvinyliques qui "collent" les cheveux et les maintiennent dans la position lisse pendant le temps de pose.

La présente invention a également pour objet un procédé d'ondulation des cheveux dans lequel on applique une composition réductrice telle que définie ci-dessus sur des cheveux mouillés préalablement roulés sur des rouleaux ayant de 4 à 20mm de diamètre, la composition pouvant éventuellement être appliquée au fur et à mesure de l'enroulage des cheveux; on laisse ensuite agir la composition réductrice pendant un temps de 5 à 60min, de préférence de 5 à 30min puis on rince abondamment après quoi on applique, sur les cheveux enroulés, une composition oxydante permettant de reformer les liaisons disulfures de la kératine pendant un temps de pose de 2 à 10min. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel ou un mélange de bromate alcalin et d'un persel. La concentration en eau oxygénée peut varier de 3 à 10 volumes, la concentration en bromate alcalin de 2 à 12% et celle

en persel de 0,1 à 15% en poids par rapport au poids total de la composition oxydante.

La présente invention a également pour objet un procédé de défrisage ou de décrépage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60min, en particulier de 5 à 30min, on procède alors à un nouveau lissage puis on rince soigneusement et on applique la composition oxydante ou fixatrice que l'on laisse agir pendant 2 à 10min environ puis on rince abondamment les cheveux.

On va maintenant donner à titre d'illustration et sans aucun caractère limitatif plusieurs exemples de préparation des N-(mercaptoalkyl) ω-hydroxyalkylamides et leurs disulfures, ainsi que plusieurs exemples de compositions les contenant.

EXEMPLE I

Préparation de l'hydroxy-4 N-(mercapto-2 éthyl) butyramide

a) Réaction réalisée à 80°C à partir de la cystéamine.

Dans un réacteur, un mélange de 15,2g de cystéamine et de 15cm$^3$ de γ-butyrolactone est agité sous atmosphère inerte pendant 5 heures à une température d'environ 80°C. La disparition de la γ-butyrolactone est suivie par chromatographie en phase vapeur (C.P.V).

Lorsque la γ-butyrolactone n'est plus détectée dans le mélange réactionnel, ce dernier est versé dans 100cm$^3$ d'eau auquel on ajoute 10g de résine sulfonique "Dowex 50" (partiellement hydratée à 45%) et 5g de poudre de zinc pour réduire la petite quantité de disulfure s'étant formée au cours de la réaction. L'ensemble est agité deux heures à température ambiante puis filtré sous atmosphère inerte. On obtient une solution aqueuse parfaitement incolore. Celle-ci est évaporée sous pression réduite et on obtient 25g d'hydroxy-4 N-(mercapto-2 éthyl) butyramide.

C'est un liquide incolore à température ordinaire.

Le spectre $^1H$ RMN 80 MHz est conforme à la structure attendue.

Le dosage des thiols en milieu acide est à 95% et l'analyse élémentaire correspondant à un produit partiellement hydraté: $C_6H_{13}NO_2S$, $1/2H_2O$

| | | | | | |
|---|---|---|---|---|---|
| Calc: | C: 41,84 | H: 8,19 | N: 8,13 | O: 23,22 | S: 18,62 |
| Tr: | 41,59 | 7,83 | 8,56 | 22,84 | 18,85 |

b) Réaction réalisée à température ambiante à partir de la cystéamine.

Dans un ballon on mélange 7,72g de cystéamine et 8,61g de γ-butyrolactone sous atmosphère inerte. Le mélange ainsi obtenu est agité pendant 10 jours à température ambiante. Le milieu initialement pâteux devient progressivement homogène pour donner un liquide limpide et relativement mobile. On vérifie à ce stade la transformation totale de la γ-butyrolactone en C.P.V et de la cystéamine par dosage à l'acide perchlorique. Le dosage du thiol est à 100%.

On obtient ainsi 15g d'hydroxy-4 N-(mercapto-2 éthyl) butyramide.

c) Réaction réalisée à partir du chlorhydrate de cystéamine.

Dans un réacteur on introduit à température ambiante, sous atmosphère inerte, 11,4g de chlorhydrate de cystéamine et goutte à goutte sous agitation 13,5cm$^3$ de triéthanolamine. Au mélange obtenu, porté à une température d'environ 60°C, on ajoute lentement 7,7cm$^3$ de γ-butyrolactone. A la fin de l'introduction l'ensemble est amené à une température de 90°C pendant trois heures, temps au bout duquel toute la γ-butyrolactone est transformée (vérification par C.P.V). On obtient ainsi 32g d'hydroxy-4 N-(mercapto-2 éthyl) butyramide contenant un équivalent de chlorhydrate de triéthanolamine sous forme d'un solide blanc pâteux que l'on conditionne sous atmosphère inerte.

Les dosages correspondent au mélange attendu.

d) Réaction réalisée à partir du chlorhydrate de cystéamine avec élimination du chlorhydrate de triéthanolamine formé

Dans un réacteur, muni d'une agitation mécanique on introduit 228g de chlorhydrate de cystéamine que l'on place sous atmosphère d'argon puis on ajoute, sous agitation, goutte à goutte, 270cm$^3$ de triéthanolamine en 1h30 environ. Le milieu réactionnel pâteux est alors porté à 60°C et à cette température on introduit goutte à goutte 155cm$^3$ de $\gamma$-butyrolactone. A la fin de l'addition, l'ensemble est alors porté pendant 8 heures à 80°C temps au bout duquel toute la $\gamma$-butyrolactone est transformée. On introduit alors 350cm$^3$ d'éthanol absolu et l'agitation est maintenue à 60°C pendant une demi-heure.

Le mélange est alors refroidi vers 15°C et le chlorhydrate de triéthanolamine est essoré. Le filtrat est concentré sous pression réduite pour éliminer l'éthanol.

On obtient 250g d'hydroxy-4 N-(mercapto-2 éthyl) butyramide.

EXEMPLE II

Préparation de l'hydroxy-5 N-(mercapto-2 éthyl) valéramide.

Un mélange, agité sous atmosphère inerte, de 5g de cystéamine et de 6,05cm$^3$ de $\delta$-valérolactone est porté à une température d'environ 60-70°C pendant 15 heures. La disparition de la $\delta$-valérolactone est suivie en C.P.V. Le mélange réactionnel est alors versé dans 100cm$^3$ d'eau et on y ajoute 10g de résine acide Dowex 50 et 3g de zinc en poudre. Le mélange obtenu est placé sous agitation pendant 4 heures à température ambiante, puis abandonné une nuit. Le lendemain on filtre et l'eau est évaporée sous pression réduite.

On obtient 8g d'hydroxy-5 N-(mercapto-2 éthyl) valéramide sous forme d'un liquide incolore dont le spectre $^1$H R.M.N ainsi que les dosages de thiol et d'amine résiduelle sont conformes à la structure attendue.

EXEMPLE III

Préparation de l'hydroxy-6 N-(mercapto-2 éthyl) caproamide.

Un mélange, sous atmosphère inerte, de 5g de cystéamine et de 7,2cm$^3$ de $\varepsilon$-caprolactone est agité pendant 6 heures à température ambiante. On abandonne une nuit puis on porte le mélange pendant 4 heures à une température d'environ 60°C, temps au bout duquel toute la $\varepsilon$-caprolactone est transformée. Le mélange est alors versé dans 100cm$^3$ d'eau auquel on ajoute 10g de résine acide Dowex 50 puis 3g de zinc en poudre et l'ensemble est agité pendant 4 heures à température ambiante. L'eau est alors éliminée sous pression réduite et on obtient 10g d'hydroxy-6 N-(mercapto-2 éthyl) caproamide sous forme d'un liquide visqueux à température ambiante dont le spectre $^1$H R.M.N et les dosages de thiol et d'amine résiduelle correspondant à la structure attendue.

EXEMPLE IV

Préparation du bis (N-éthyl hydroxy-4 butyramide) disulfure 1ère méthode

Une solution de 20 g d'hydroxy-4 N-(mercapto-2 éthyl) butyramide dans 300 cm$^3$ d'éthanol anhydre est agitée à l'air en présence de 20 cm$^3$ de triéthylamine pendant 48 heures, temps au bout duquel le thiol est transformé en disulfure correspondant. Le solvant et la triéthylamine sont éliminés par évaporation sous vide.

Le produit pâteux obtenu est divisé par agitation dans le chlorure de méthylène, essoré, puis recristallisé dans 200 cm$^3$ d'acétanitrile.

On obtient 15 g de bis (N-éthyl hydroxy-4 butyramide) disulfure sous forme de cristaux blancs de point de fusion : 102°C.

Analyse élémentaire : $C_{12}H_{24}N_2O_4S_2$

| | C | H | N | O | S |
|---|---|---|---|---|---|
| Calc. | 44,42 | 7,47 | 8,64 | 19,72 | 19,76 |
| Tr. | 44,55 | 7,51 | 8,47 | 20,02 | 19,67 |

2ème méthode

Dans un réacteur on introduit, successivement et à température ambiante, 11,26 g (0,05 mole) de chlorhydrate de cystamine, 14,92 g (0,1 mole) de triéthanolamine, 8,61 g (0,1 mole) de γ-butyrolactone et enfin 20 $cm^3$ d'éthanol absolu. Le mélange ainsi obtenu est porté 4 heures à 80°C.

Le milieu réactionnel est essoré sur verre fritté. On traite séparément le solide et le filtrat.

Le solide est repris avec 75 $cm^3$ d'acétonitrile à ébullition. L'insoluble est éliminé par filtration et le filtrat conduit par refroidissement à 8,3 g d'un solide blanc.

Le filtrat est évaporé à sec puis repris avec 30 $cm^3$ d'acétonitrile à ébullition. L'insoluble est éliminé sur verre fritté et le filtrat donne par refroidissement 2,4 g d'un solide blanc.

On obtient au total 10,7 g de bis (N-éthyl hydroxy-4 butyramide) disulfure de point de fusion : 99-100°C.

Le spectre RMN'H 80 MHz est conforme à la structure attendue.

Selon le même mode opératoire que décrit ci-dessus, on a également obtenu les autres disulfures mentionnés à la page 5.

EXEMPLE V

Préparation du bis (N-éthyl hydroxy-5 valéramide) disulfure

A une solution de 1,77 g (0,1 mole) d'hydroxy-5 N-(mercapto-2 éthyl) valéramide dans 20 $cm^3$ d'alcool absolu, refroidie à + 5° C, on ajoute goutte à goutte 4,6 $cm^3$ (0,045 mole) d'eau oxygénée à 30 %.

L'agitation est ensuite maintenue 1 heure en laissant revenir à température ambiante. Le dosage des thiols est négatif. Le milieu réactionnel est alors évaporé à sec sous pression réduite. Après séchage prolongé sous vide à température ambiante, on obtient 1,5 g de bis (N-éthyl hydroxy-5 valéramide) disulfure qui cristallise lentement sous la forme d'un solide cireux blanc.

Le spectre RMN'H 80 MHz est conforme à la structure attendue.

EXEMPLE VI

Préparation du bis (N-éthyl hydroxy-6 caproamide) disulfure

A une solution de 1,92 g (0,1 mole) d'hydroxy-6 N-(mercapto-2 éthyl) caproamide dans 20 $cm^3$ d'éthanol, refroidie à + 5° C, on ajoute goutte à goutte sous agitation 5,0 $cm^3$ (0,049 mole) d'eau oxygénée à 30 %. Après 2 heures d'agitation à température ambiante (test des thiols négatif) la solution est clarifiée par filtration sur papier puis évaporée à sec sous pression réduite. Après séchage prolongé sous vide à température ambiante, on obtient 1,7g de bis (N-éthyl hydroxy-6 caproamide) disulfure sous la forme d'un gel épais jaune pâle.

Le spectre RMN'H 80 MHz est conforme à la structure attendue.

EXEMPLES DE COMPOSITION

I - On prépare, selon l'invention, une composition réductrice de déformation permanente des cheveux en procédant au mélange des ingrédients suivants:

| | |
|---|---|
| - Hydroxy-4 N-(mercapto-2 éthyl) butyramide...... | 9g |
| - Ammoniaque qsp.......................... | pH 9 |
| - Parfum........................................ | 0,1g |
| - Conservateur................................ | 0,5g |
| - Eau déminéralisée qsp...................... | 100g |

Cette composition est appliquée sur des cheveux mouillés préalablement enroulés sur des rouleaux de mise en plis. Après avoir laissé agir la composition pendant environ 15min on rince abondamment à l'eau puis on applique la composition oxydante suivante:

- Eau oxygénée q.s.p............................ 8 volumes
- Stabilisant.................................. 0,3g
- Parfum....................................... 0,1g
- Acide lactique qsp.................... pH 3
- Eau déminéralisée qsp........................ 100g

On laisse agir la composition oxydante pendant environ 10 min puis on enlève les rouleaux et rince abondamment la chevelure à l'eau.

Après séchage sous casque les cheveux présentent de belles boucles.

Dans cet exemple la composition réductrice peut être avantageusement remplacée par l'une des compositions réductrices suivantes :

A) - Hydroxy-4 N-(mercapto-2 éthyl butyramide) .... 12g
- Monoéthanolamine qsp ............. pH 9
- Parfum ...................................... 0,1g
- Conservateur ................................ 0,5g
- Eau déminéralisée qsp ....................... 100g

B) - Hydroxy-5 N-(mercapto-2 éthyl) valéramide .... 10g
- Monoéthanolamine qsp ............. pH 8,5
- Parfum ...................................... 0,1g
- Conservateur ................................ 0,5g
- Eau déminéralisée qsp ....................... 100g

C) - Hydroxy-6 N-(mercapto-2 éthyl) caproamide .... 7g
- Ammoniaque qsp .................. pH 8,5
- Parfum ...................................... 0,1g
- Conservateur ................................ 0,5g
- Eau déminéralisée qsp ....................... 100g

II- On prépare, selon l'invention, une composition pour effectuer une permanente dite "auto-neutralisante" en mélangeant les composés suivants :

- Hydroxy-4 N-(mercapto-2 éthyl)butyramide ........ 6,3g
- Bis-(N-éthyl hydroxy-4 butyramide) disulfure ....17,5g
- Urée ............................................ 8 g
- Parfum .......................................... 0,1g
- Conservateur .................................... 0,5g
- Ammoniaque qsp ............................. pH 9,5
- Eau qsp ...................................... 100g

On applique cette solution sur des cheveux mouillés préalablement enroulés sur des rouleaux de mise en plis. On observe un temps de pause d'environ 20 minutes puis on enlève les rouleaux et on rince abondamment

les cheveux à l'eau.

Après séchage sous casque la chevelure présente de belles ondulations.

## Revendications

1. N-(mercaptoalkyl) ω-hydroxyalkylamides, caractérisés par le fait qu'ils correspondent à la formule générale suivante:

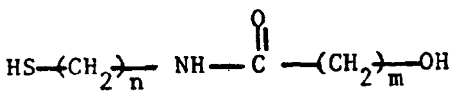

(I)

dans laquelle:

n est 2 ou 3, et

m est 2 à 5 inclus.

et leurs disulfures correspondants.

2. Composés selon la revendication 1, caractérisés par le fait que n est 2 et m est 3 ou 4.

3. Composés selon la revendication 1 ou 2, caractérisés par le fait qu'ils sont:

   l'hydroxy-4 N-(mercapto-2 éthyl) butyramide,
   l'hydroxy-3 N-(mercapto-2 éthyl) propionamide,
   l'hydroxy-5 N-(mercapto-2 éthyl) valéramide,
   l'hydroxy-4 N-(mercapto-3 propyl) butyramide,
   l'hydroxy-6 N-(mercapto-2 éthyl) caproamide, et
   l'hydroxy-6 N-(mercapto-3 propyl) caproamide.

4. Procédé de préparation des N-(mercaptoalkyl) ω-hydroxyalkylamides selon les revendications 1 à 3, caractérisé par le fait qu'il consiste à faire réagir une mercaptoalkylamine de formule: $HS-(CH_2)_n-NH_2$ sur une lactone de formule :

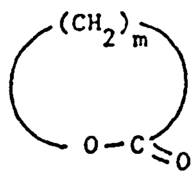

dans lesquelles: n est 2 ou 3, et m est 2 à 5 inclus, la réaction étant conduite, avec ou sans solvant, à une température comprise entre 20 et 110°C.

5. Procédé selon la revendication 4, caractérisé par le fait que la réaction est réalisée en présence d'un alcool pris dans le groupe constitué par le méthanol, l'éthanol, l'isopropanol et le butanol.

6. Procédé selon l'une quelconque des revendications 4 et 5, caractérisé par le fait que la mercaptoalkylamine est la mercapto-2 éthylamine ou la mercapto-3 propylamine.

7. Procédé selon l'une quelconque des revendications 4 et 5, caractérisé par le fait que la lactone est la β-propionolactone, la γ-butyrolactone, la valérolactone ou la caprolactone.

8. Composés selon la revendication 1 sous forme de leurs disulfures ayant la formule générale suivante :

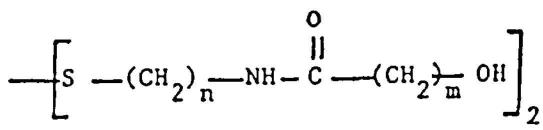

dans laquelle :

n et m sont tels que définis à la revendication 1.

**9.** Composés selon la revendication 8 caractérisés par le fait qu'ils sont :

le bis (N-éthyl hydroxy-4 butyramide) disulfure,
le bis (N-éthyl hydroxy-5 valéramide) disulfure,
le bis (N-éthyl hydroxy-6 caproamide) disulfure,
le bis (N-éthyl hydroxy-3 propionamide) disulfure,
le bis (N-propyl hydroxy-4 butyramide) disulfure, et
le bis (N-propyl hydroxy-6 caproamide) disulfure.

**10.** Composition cosmétique réductrice pour le premier temps d'une opération de déformation permanente des cheveux, caractérisée par le fait qu'elle contient, dans un véhicule cosmétique approprié, en tant qu'agent réducteur, au moins un N-(mercaptoalkyl) ω-hydroxyalkylamide de formule (I) selon l'une quelconque des revendications 1 à 3, ou obtenu selon l'une quelconque des revendications 4 à 7.

**11.** Composition selon la revendication 10, caractérisée par le fait que l'agent réducteur est présent à une concentration comprise entre 2 et 20% en poids et de préférence entre 5 et 10% en poids par rapport au poids total de la composition.

**12.** Composition selon l'une quelconque des revendications 10 et 11, caractérisée par le fait que son pH est compris entre 6,5 et 10.

**13.** Composition selon l'une quelconque des revendications 10 à 12, caractérisée par le fait qu'elle contient en outre au moins un polymère cationique, un agent adoucissant, un hydrolysat de protéine, une cire, un agent opacifiant, un parfum, un colorant, un tensio-actif non-ionique ou cationique, un agent traitant ou un agent de pénétration.

**14.** Procédé de déformation permanente des cheveux consistant dans une première étape à réduire les liaisons disulfures de la kératine par application d'une composition réductrice puis dans une seconde étape à reformer lesdites liaisons par application d'une composition oxydante, caractérisé par le fait que l'étape de réduction est réalisée à l'aide d'une composition cosmétique réductrice telle que revendiquée selon l'une quelconque des revendications 10 à 13.

**15.** Procédé selon la revendication 14, caractérisé par le fait que l'on laisse agir la composition réductrice pendant un temps compris entre 5 et 60 min.

## Patentansprüche

**1.** N-(Mercaptoalkyl-) ω-hydroxyalkylamide, dadurch gekennzeichnet, daß sie der folgenden allgemeinen Formel entsprechen:

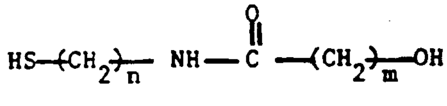

(I)

in der

n 2 oder 3 ist,

und

m 2 bis einschließlich 5 ist,

und ihre entsprechenden Disulfide.

2. Verbindungen nach Anspruch 1, dadurch gekennzeichnet, daß n 2 ist und m 3 oder 4 ist.

3. Verbindungen nach Anspruch 1 oder 2, dadurch gekennzeichnet, daß es sich um die folgenden Verbindungen handelt:
   4-Hydroxy-N-(2-mercaptoethyl-)butyramid;
   3-Hydroxy-N-(2-mercaptoethyl-)propionamid;
   5-Hydroxy-N-(2-mercaptoethyl-)valeramid;
   4-Hydroxy-N-(3-mercaptopropyl-)butyramid;
   6-Hydroxy-N-(2-mercaptoethyl-)caproamid;
   und
   6-Hydroxy-N-(3-mercaptopropyl-)caproamid.

4. Verfahren zur Herstellung von N-(Mercaptoalkyl-)ω-hydroxyalkylamiden gemäß den Ansprüchen 1 bis 3, dadurch gekennzeichnet, daß es aus einer Umsetzung eines Mercaptoalkylamins der Formel $HS-(CH_2)_n-NH_2$ mit einem Lacton der Formel

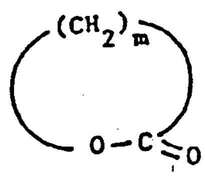

besteht, worin n 2 oder 3 ist und m 2 bis einschließlich 5 ist, wobei die Reaktion mit oder ohne Lösungsmittel bei einer Temperatur im Bereich von 20 bis 110°C durchgeführt wird.

5. Verfahren nach Anspruch 4, dadurch gekennzeichnet, daß die Reaktion in Gegenwart eines Alkohols aus der aus Methanol, Ethanol, Isopropanol und Butanol bestehenden Gruppe durchgeführt wird.

6. Verfahren nach irgendeinem der Ansprüche 4 und 5, dadurch gekennzeichnet, daß das Mercaptoalkylamin 2-Mercaptoethylamin oder 3-Mercaptopropylamin ist.

7. Verfahren nach irgendeinem der Ansprüche 4 und 5, dadurch gekennzeichnet, daß das Lacton β-Propiolacton, γ-Butyrolacton, Valerolacton oder Caprolacton ist.

8. Verbindungen nach Patentanspruch 1 in Form der Disulfide, die die folgende allgemeine Formel aufweisen:

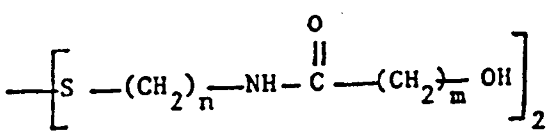

in der n und m wie in Patentanspruch 1 definiert sind.

9. Verbindungen nach Anspruch 8, dadurch gekennzeichnet, daß es sich um die folgenden Verbindungen handelt:
   Bis-(N-ethyl-4-hydroxybutyramid-)disulfid;
   Bis-(N-ethyl-5-hydroxyvaleramid-)disulfid;
   Bis-(N-ethyl-6-hydroxycaproamid-)disulfid;
   Bis-(N-ethyl-3-hydroxypropionamid-)disulfid;
   Bis-(N-propyl-4-hydroxybutyramid-)disulfid;
   und
   Bis-(N-propyl-6-hydroxycaproamid-)disulfid.

10. Für die erste Zeit eines Verfahrens zur bleibenden Verformung der Haare reduzierende kosmetische Zubereitung, dadurch gekennzeichnet, daß sie in einem geeigneten kosmetischen Träger als Reduktionsmittel wenigstens ein N-(Mercaptoalkyl-)ω-hydroxyalkylamid der Formel (I) gemäß irgendeinem der Ansprüche 1 bis 3 oder erhalten gemäß irgendeinem der Ansprüche 4 bis 7 enthält.

**11.** Zubereitung nach Anspruch 10, dadurch gekennzeichnet, daß das Reduktionsmittel in einer Konzentration im Bereich von 2 bis 20 Gew.-% und bevorzugt im Bereich von 5 bis 10 Gew.-%, bezogen auf das Gesamtgewicht der Zubereitung, zugegen ist.

**12.** Zubereitung nach irgendeinem der Ansprüche 10 und 11, dadurch gekennzeichnet, daß ihr pH-Wert im Bereich zwischen 6,5 und 10 liegt.

**13.** Zubereitung nach irgendeinem der Ansprüche 10 bis 12, dadurch gekennzeichnet, daß sie außerdem wenigstens ein kationisches Polymer, einen Weichmacher, ein Protein-Hydrolysat, ein Wachs, ein opazifizierendes Mittel, ein Parfum, ein Färbemittel, ein nichtionisches oder kationische oberflächenaktives Mittel, ein Behandlungsmittel oder ein Durchdringungsmittel enthält.

**14.** Verfahren zur bleibenden Verformung der Haare, welches in einem ersten Schritt aus einer Reduktion der Disulfid-Bindungen des Keratins durch Anwendung einer reduzierenden Zubereitung und danach in einem zweiten Schritt aus einer erneuten Bildung der Bindungen durch Anwendung einer oxidierenden Zubereitung besteht, dadurch gekennzeichnet, daß der Reduktionsschritt mit Hilfe einer reduzierenden kosmetischen Zubereitung durchgeführt wird, wie sie in irgendeinem der Ansprüche 10 bis 13 beansprucht ist.

**15.** Verfahren nach Anspruch 14, dadurch gekennzeichnet, daß man die reduzierende Zubereitung während einer Zeit im Bereich von 5 bis 60 Minuten einwirken läßt.

## Claims

**1.** N-(mercaptoalkyl)-ω-hydroxyalkylamides, characterised in that they correspond to the following general formula:

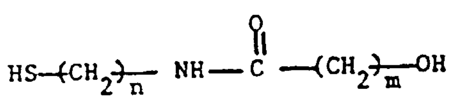

(I)

in which:
n is 2 or 3, and
m is 2 to 5 inclusive,
and their corresponding disulphides.

**2.** Compounds according to Claim 1, characterised in that n is 2 and m is 3 or 4.

**3.** Compounds according to Claim 1 or 2, characterised in that they are:
4-hydroxy-N-(2-mercaptoethyl)butyramide,
3-hydroxy-N-(2-mercaptoethyl)propionamide,
5-hydroxy-N-(2-mercaptoethyl)valeramide,
4-hydroxy-N-(3-mercaptopropyl)butyramide,
6-hydroxy-N-(2-mercaptoethyl)caproamide, and
6-hydroxy-N-(3-mercaptopropyl)caproamide.

**4.** Process for preparing N-(mercaptoalkyl)-ω-hydroxyalkylamides according to Claims 1 to 3, characterised in that it consists in reacting a mercaptoalkylamine of formula: $HS-(CH_2)_n-NH_2$
with a lactone of formula:

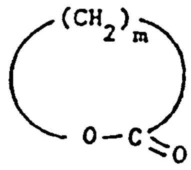

in which formulae: n is 2 or 3, and m is 2 to 5 inclusive,
the reaction being carried out, with or without solvent, at a temperature of between 20 and 110°C.

5. Process according to Claim 4, characterised in that the reaction is carried out in the presence of an alcohol which is chosen from the group consisting of methanol, ethanol, isopropanol and butanol.

6. Process according to either of Claims 4 and 5, characterised in that the mercaptoalkylamine is 2-mercaptoethylamine or 3-mercaptopropylamine.

7. Process according to either of Claims 4 and 5, characterised in that the lactone is β-propionolactone, γ-butyrolactone, valerolactone or caprolactone.

8. Compounds according to Claim 1, in the form of their disulphides of the following general formula:

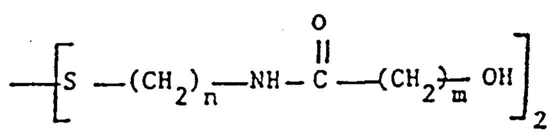

in which:
n and m are as defined in Claim 1.

9. Compounds according to Claim 8, characterised in that they are:
bis(N-ethyl-4-hydroxybutyramide) disulphide,
bis(N-ethyl-5-hydroxyvaleramide) disulphide,
bis(N-ethyl-6-hydroxycaproamide) disulphide,
bis(N-ethyl-3-hydroxypropionamide) disulphide,
bis(N-propyl-4-hydroxybutyramide) disulphide, and
bis(N-propyl-6-hydroxycaproamide) disulphide.

10. Reducing cosmetic composition for the first stage of an operation for the permanent deformation of hair, characterised in that it contains, in a cosmetically appropriate vehicle, as reducing agent, at least one N-(mercaptoalkyl)-ω-hydroxyalkylamide of formula (I) according to any one of Claims 1 to 3 or obtained according to any one of Claims 4 to 7.

11. Composition according to Claim 10, characterised in that the reducing agent is present at a concentration of between 2 and 20% by weight, and preferably between 5 and 10% by weight, relative to the total weight of the composition.

12. Composition according to either of Claims 10 and 11, characterised in that its pH is between 6.5 and 10.

13. Composition according to any one of Claims 10 to 12, characterised in that it contains, in addition, at least one cationic polymer, one demulcent, one protein hydrolysate, one wax, one opacifying agent, one perfume, one colorant, one nonionic or cationic surface-active agent, one treatment agent or one penetrating agent.

14. Process for the permanent deformation of hair consisting, in a first stage, in reducing the disulphide bonds of keratin by applying a reducing composition and then, in a second stage, in re-forming the said bonds by applying an oxidising composition, characterised in that the reduction stage is carried out by means of a reducing cosmetic composition as claimed according to any one of Claims 10 to 13.

15. Process according to Claim 14, characterised in that the reducing composition is allowed to act for a period of between 5 and 60 min.